(19) **Europäisches Patentamt / European Patent Office / Office européen des brevets**

(11) **EP 1 372 465 B1**

(12) **EUROPÄISCHE PATENTSCHRIFT**

(45) Veröffentlichungstag und Bekanntmachung des Hinweises auf die Patenterteilung:
**27.06.2007 Patentblatt 2007/26**

(21) Anmeldenummer: **01929366.1**

(22) Anmeldetag: **27.02.2001**

(51) Int Cl.:
*A61B 5/00* (2006.01)      *A61B 5/053* (2006.01)

(86) Internationale Anmeldenummer:
**PCT/EP2001/002194**

(87) Internationale Veröffentlichungsnummer:
**WO 2002/067774 (06.09.2002 Gazette 2002/36)**

(54) **SONDE FÜR DIE DIELEKTRISCHE UND OPTISCHE DIAGNOSTIK**

PROBE FOR DIELECTRIC AND OPTICAL DIAGNOSIS

SONDE POUR LE DIAGNOSTIC DIELECTRIQUE ET OPTIQUE

(84) Benannte Vertragsstaaten:
**AT BE CH CY DE DK ES FI FR GB GR IE IT LI LU MC NL PT SE TR**

(43) Veröffentlichungstag der Anmeldung:
**02.01.2004 Patentblatt 2004/01**

(73) Patentinhaber: **Fraunhofer-Gesellschaft zur Förderung der angewandten Forschung e.V. 80686 München (DE)**

(72) Erfinder: **SCHRAMM, Werner 15370 Fredersdorf (DE)**

(56) Entgegenhaltungen:
**DE-A- 19 854 292      GB-A- 1 470 890**

EP 1 372 465 B1

**Beschreibung**

Die Erfindung betrifft eine endoskopische Messsonde für die medizinische Diagnostik, mit der dielektrische und optische Parameter am gleichen Messort erfasst werden können. Anwendungsgebiet ist die Tumordiagnostik, bei der sowohl auf der Gewebeoberfläche als auch durch Eindringen in das Gewebe gemessen wird.

Es sind einige Messanordnungen bekannt, mit denen Mehrfach-Messungen am Gewebe durchgeführt werden, um Tumorgewebe von gesundem Gewebe zu unterscheiden.

In US 5 800 350 wird zum Beispiel eine Messsonde beschrieben, die mit dem Gewebe in Kontakt gebracht wird und es ermöglicht, eine Vielzahl von unterschiedlichen physikalischen Meßgrößen des Gewebes, u.a. auch elektrische und optische zu erfassen.

Die Elektroden für die elektrischen Messungen, die auf der Stirnseite der Messsonde liegen, sind so gestaltet und angeordnet, dass nach Aufsetzen der Sonde auf das Gewebe unterschiedliche Widerstandsstrecken zwischen einer Mittelelektrode und separaten Elektrodensegmenten an der Peripherie vergleichend ausgemessen werden, um einen reproduzierbaren Sitz der Sonde auf dem Gewebe sicherzustellen. Es können auch Abklingzeiten von Stromimpulsen, die durch das Gewebe geschickt werden, ausgewertet werden.

Zur Erfassung der optischen Gewebeeigenschaften, für die die Wellenlängen 540, 650, 660, 940 und 1300 nm als von hohem diagnostischen Wert angesehen werden, sind in der Sonde Lichtleitfasern vorhanden, die das Licht von den Lichtquellen zum Gewebe oder von diesem zurück zu entsprechenden Empfängern transportieren.

In DE 198 54 292 ist eine Multiparametermessanordnung vorgeschlagen worden, bei der die verwendete Messsonde einen separaten Koaxialleitungstrakt und seitlich daneben diverse Lichtleitfasern für die optische Spektroskopie aufweist.

In beiden erwähnten Messanordnungen ist nachteilig, dass der Messort für die Impedanzmessung nicht genau mit dem für die optisch-spektroskopischen Messungen übereinstimmt.

Aufgabe der Erfindung ist es deshalb, eine Anordnung anzugeben, mit der bei einer Messung an einer bestimmten Stelle des Gewebes zugleich sowohl dielektrische als auch optische Parameter erfasst werden können.

Die Aufgabe wird mit der Anordnung gemäß dem Patentanspruch 1 gelöst. Vorteilhafte Ausgestaltungen sind Gegenstand der Unteransprüche.

Durch die erfindungsgemäße Kombination von elektrischem Wellenleiter mit optischen Lichtleitfasern in einer Messsonde gelingt es, mehrere diagnostische Parameter genau am gleichen Gewebeareal zu erfassen, was für die sichere Unterscheidung von Tumor und gesundem Gewebe von entscheidender Bedeutung ist. Dabei ist der geringe Platzbedarf vorteilhaft für die Herstellbarkeit sehr dünner endoskopischer Meßsonden.

Die Erfindung wird anhand von Ausführungsbeispielen näher erläutert.

In den Zeichnungen zeigen:

Fig. 1: Prinzipdarstellung einer Messanordnung, bei der die endoskopische Messsonde über einen Kopplungsmodul mit einem Betriebsgerät verbunden ist.
Fig. 2: Verteilung von Lichtleitfasern mit unterschiedlicher Funktion über den Querschnitt einer Koaxialanordnung
Fig. 3: Einsatzbeispiele für Messungen im Kontakt mit dem Gewebe
3a invasiv
3b nichtinvasiv, aufsitzend

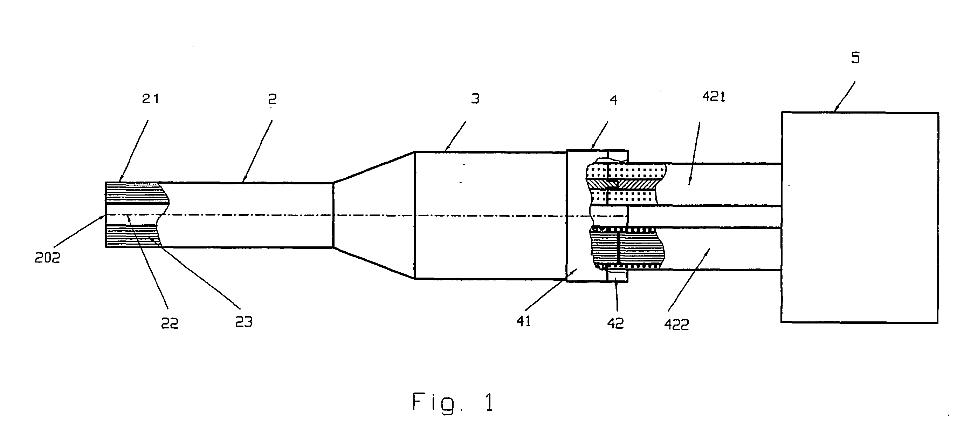

**Fig.1** zeigt den prinzipiellen Aufbau einer Meßanordnung für die ortsgenaue dielektrische und optisch-spektroskopische Diagnostik von biologischem Gewebe, wobei neben der zylindrischen endoskopischen Messsonde 2 mit Schaft 3 und Kopplungsmodul 4 das Bedienungsgerät 5 dargestellt wird, das über ein Koaxialkabel 421 und ein Lichtleiterkabel 422 angeschlossen ist.

Die Messsonde 2 ist als Kombination von elektrischem Wellenleiter und Lichtleitermesssonde ausgebildet. Der Raum zwischen dem metallischen Außenmantel 21 und dem koaxialen Innenleiter 22 ist mit einem Dielektrikum aus Lichtleitfasern 23 ausgefüllt. Der Wellenwiderstand z dieser Anordnung wird zweckmäßigerweise so gewählt, dass er mit dem Wellenwiderstand des über den Kopplungsmodul 4 angeschlossenen Koaxialkabels 421 übereinstimmt.

Der Wellenwiderstand z wird in bekannter Weise (Meinke, Grundlach, Handbuch der Hochfrequenztechnik, 3. Auflage, Springer 1968, S. 255) aus dem Durchmesser D des Außenmantels 21, dem Durchmesser d des Innenleiters 22 und der Dielektrizitätskonstante $\varepsilon_r$ des Dielektrikums 23 berechnet nach der Formel:

$$z = 60 \, / \varepsilon_r^{-1/2} \ln D/d$$

Die Messsonde 2 geht an ihrem distalen Ende in einen Schaft 3 über, an den sich ein aus Steckerteil 41 und Buchsenteil 42 bestehender Kopplungsmodul 4 anschließt, der seinerseits über ein Koaxialkabel 421 und Lichtleiterkabel 422 mit dem für den Betrieb der Messsonde notwendigen Bedienungsgerät 5 verbunden ist. Dieses enthält die Signalquellen und die Signalempfänger für die dielektrische und optische Spektroskopie,

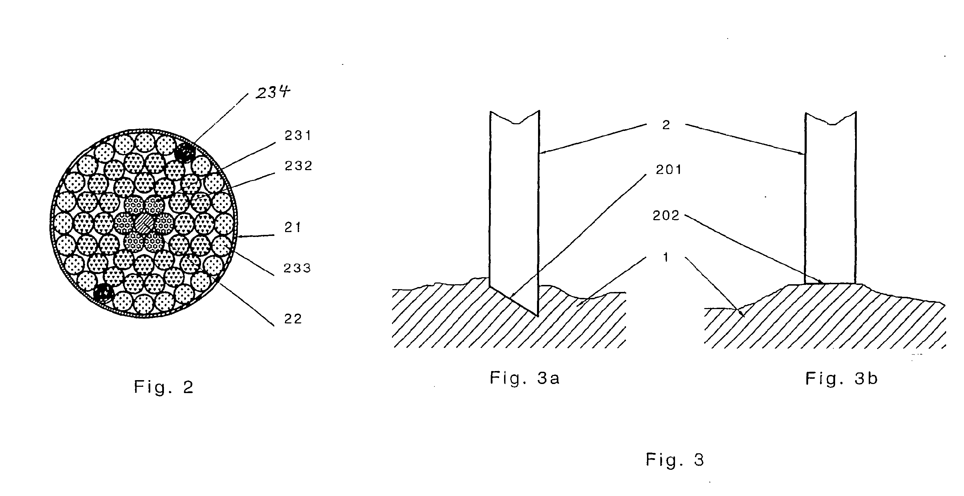

In **Fig.2** ist der Querschnitt einer erfindungsgemäßen Messsonde dargestellt, die Lichtleitfasern unterschiedlicher Funktion aufweist. Dabei sind die Lichtleitfasern gleichmäßig über den Querschnitt verteilt, um den Charakter eines symmetrischen Wellenleiters sicherzustellen. Mit 231 werden Anregungsfasern bezeichnet, die Anregungslicht von z.B. 337 nm zum Gewebe bringen, mit 232 Fasern, die für die Messung der Fluoreszenz des Gewebes vorgesehen sind, mit 233 Fasern, die die Streuung des Anregungslichtes im Gewebe erfassen, mit 234 Fasern für die Oberflächen-Ramanspektroskopie.

**Fig. 3** veranschaulicht zwei Anwendungsfälle für erfindungsgemäß unterschiedlich ausgebildete Stirnseiten der Messsonde 2. In **Fig. 4a** ist eine Endoskopmesssonde 2 mit einer als Spitze ausgeführten Stirnseite 201 gezeigt, die für eine invasive Messung im Gewebe 1 benutzt wird. **Fig. 4b** stellt den Anwendungsfall dar, dass die Messsonde 2 eine ebene Stirnseite 202 besitzt und zur Messung auf das Gewebe 1 aufgesetzt ist.

**Aufstellung der Bezugszeichen**

1 Gewebe

2 Messsonde
201 als Spitze ausgeführte Stirnseite
202 ebene Stirnseite
21 metallischer Außenmantel
22 metallischer Innenleiter
23 Lichtleitfasern
231 Anregungsfasern
232 Fluoreszenzmeßfasern
233 Remissionsmeßfasern
234 Fasern für Oberflächen-Ramanspektroskopie

3 Schaft der endoskopischen Messsonde

4 Kopplungsmodul
41 Steckerteil
42 Buchsenteil
421 Koaxialkabel
422 Lichtleiterkabel

5 Gerät zum Betrieb der Messsonde

**Patentansprüche**

1. Endoskopische Messsonde für die Multiparameterdiagnostik von biologischem Gewebe, **dadurch gekennzeichnet, dass** ein als koaxialer Wellenleiter ausgebildeter zylinderförmiger Messkopf (2), der mit dem zu untersuchenden Gewebe (1) in Kontakt gebracht wird, einen metallischen Außenmantel (21) und einen koaxialen Innenleiter (22) aufweist, wobei der Raum zwischen beiden durch ein Dielektrikum aus Lichtleitfasern (23) ausgefüllt wird, die gruppenweise oder auch einzeln Licht bestimmter Wellenlänge von Signalquellen zum Gewebe oder von diesem weg zu optischen Empfängern übertragen, wobei dieser Messkopf an seinem distalen Ende in einen Schaft (3) mit Kopplungsmodul (4) ausläuft, der aus Steckerteil (41) und Buchsenteil (42) bestehend eine lösbare Trennstelle zum Bedienungsgerät (5) darstellt.

2. Endoskopische Messsonde nach Anspruch 1, **dadurch gekennzeichnet, dass** die Lichtleitfasern als Dielektrikum gleichmäßig über den Querschnitt verteilt sind.

3. Endoskopische Messsonde nach Anspruch 1 und 2, **dadurch gekennzeichnet, dass** ihr proximales Ende für das Eindringen in das Gewebe (1) als Spitze (201) ausgebildet ist.

4. Endoskopische Messsonde nach Anspruch 1 und 2, **dadurch gekennzeichnet, dass** ihr proximales Ende eine ebene Stirnseite (202) aufweist.

**Claims**

1. An endoscopic measuring probe for multi-parameter diagnostics of biological tissues, **characterized in that** a cylindrical measuring head (2) formed as a coaxial waveguide, which is brought into contact with the tissue (1) to be investigated, comprises a metallic outer sheath (21) and a coaxial inner conductor (22), wherein the space between the two is filled by a dielectric of optical fibres (23), which in groups or singly transmit light of specific wavelengths from signal sources to the tissue or back from this to optical receivers, wherein this measuring head (2) terminates at its distal end in a shank (3) with a coupling module (4) which provides a releasable connecting point to the operating apparatus (5), consisting of a plug part (41) and socket part (42).

2. An endoscopic measuring probe according to claim 1, **characterized in that** the optical fibres are distributed uniformly over the cross-section as dielectric.

3. An endoscopic measuring probe according to claim 1 or 2, **characterized in that** the optical fibres are formed at their proximal end as a point (201) for penetration into the tissue.

4. An endoscopic measuring probe according to claim 1 or 2, **characterized in that** it has a flat end (202) at its proximal end.

**Revendications**

1. Sonde de mesure endoscopique pour un diagnostic multi-paramètres de tissues biologiques, **caractérisée par**

une tête de mesure (2), cylindrique, réalisée comme guide d'ondes coaxial, que l'on met en contact avec le tissu (1) à examiner, une enveloppe extérieure (21) métallique et un conducteur intérieur (22) coaxial, l'espace entre les deux étant remplie par un diélectrique formé de fibres guides de lumière (23) qui transmettent par groupes ou séparément de la lumière d'une longueur d'onde définie fournie par des sources de signaux vers le tissu et à partir de celui-ci vers des récepteurs optiques, cette tête de mesure se terminant à son extrémité distale par une tige (3) munie d'un module de couplage (4) composé d'une partie de connecteur (41) et d'une partie de douille (42) constituant un point de liaison séparable avec l'appareil de service (5).

2.  Sonde de mesure endoscopique selon la revendication 1,
    **caractérisée en ce que**
    les fibres guides de lumière sont réparties comme diélectrique, régulièrement sur la section.

3.  Sonde de mesure endoscopique selon la revendication 1 ou 2,
    **caractérisée en ce que**
    son extrémité proximale est en forme de pointe (201) pour pénétrer dans le tissu (1).

4.  Sonde selon les revendications 1 ou 2,
    **caractérisée en ce que**
    l'extrémité proximale a une face frontale (202) plane.

Fig. 1

Fig. 3a    Fig. 3b

Fig. 3

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5800350 A
- DE 19854292

**In der Beschreibung aufgeführte Nicht-Patentliteratur**

- **MEINKE ; GRUNDLACH.** *Handbuch der Hochfrequenztechnik,* 1968, vol. 3, 255